# EUROPEAN PATENT SPECIFICATION

(11) **EP 0 526 540 B1**
(45) Date of publication and mention of the grant of the patent: **30.06.1999**
(21) Application number: 91908623.1
(22) Date of filing: 24.04.1991
(51) Int. Cl.: A61K 31/00, A61K 31/46

(54) **USE OF 5-HT4-RECEPTOR ANTAGONISTS IN THE TREATMENT OF ATRIAL FIBRILLATION, INCLUDING THE PREVENTION OF STROKE**
VERWENDUNG VON 5-HT4 REZEPTORANTAGONISTEN ZUR BEHANDLUNG VON VORHOF-FIBRILLATION UND ZUR VORBEUGUNG VON SCHLAGANFALL
UTILISATION D'ANTAGONISTES DE RECEPTEURS 5-HT4 DANS LE TRAITEMENT DE LA FIBRILLATION AURICULAIRE, EN PARTICULIER LA PREVENTION DE L'APOPLEXIE

(30) Priority: 26.04.1990 GB 9009389
(43) Date of publication of application: 10.02.1993
(73) Proprietor: SMITH KLINE & FRENCH LABORATORIES LIMITED, Welwyn Garden City Hertfordshire, AL7 1EY (GB); BEECHAM GROUP PLC, Brentford, Middlesex TW8 9EP (GB)
(72) Inventor: KAUMANN, Alberto Julio, Welwyn Hertfordshire AL6 9AR (GB); King, Francis David, The Pinnacles Harlow Essex CM19 5AD (GB)
(74) Representative: Giddings, Peter John, Dr.
(86) International application number: GB9100650
(87) International publication number: WO9116045

(56) References cited:
- Journal of Medicinal Chemistry, vol. 30, no. 9, September 1987, American Chemical Society, US; P. Fludzinski et al.: "Indazoles as indole bioiososteres: synthesis and evaluation of the tropanyl ester and amide of indazole-3-carboxylate as antagonists at the serotonin 5HT3 receptor", pages 1535-1537, see the whole article
- Br. J. Pharmacol, vol. 98, October 1989, A.J. Kaumann et al.: "A receptor for 5-HT in human atrium", page 664P, see the whole article (cited in the application)
- J. Cardiovasc. Pharmacol., vol. 7, suppl. 7, 1985, Raven Press, New York, US; A.J. Kaumann: "Two classes of myocardial 5-hydroxytryptamine receptors that are neither 5-HT, Nor 5-HT2", pages S76-S78, see summary; page S78, right-hand column, last paragraph
- J. Physiol., vol. 435, April 1991, H. Ouadid et al.: "5-hydroxytryptamine increases Ca current in single human atrial cells via a 5-HT4, receptor positively coupled to adenylate cyclase", page 93P, see the whole article
- J. Cardiovasc. Pharmacol., vol. 15, suppl. 7, 1990, Raven Press Ltd, New York, US; (Int. Symposium, Bonn, November 18, 1989), P.R. Saxena et al.: "Cardiovascular effects of serotonin agonists and antagonists", pages S17-S34, see summary; page S29, left-hand column, last paragraph
- Eur. J. Pharmacol., vol. 183, no. 4, 4 July 1990, XIth Int. Congress of Pharmacology, 1-6 July 1990, Amsterdam, NL; C.M. Villal n et al.: "Putative 5-HT4 receptor mediates 5-hydroxytryptamine-induced tachycardia in the anaesthetized pig", page 1160, see the whole abstract
- Br. J. Pharmacol., vol. 102, no. 1, January 1991, Macmillan Press, Ltd, C.M. Villal n et al.: "Further characterization by use of tryptamine and benzamide derivates, of the putative 5-HT4 receptor mediating tachycardia in the pig", pages 107-112, see the whole article
- Naunyn Schmiedeberg's Arch. Pharmacol., vol. 342, no. 5, November 1990, Springer-Verlag, A.J. Kaumann: "Piglet sinoatrial 5-HT receptors resemble human atrial 5-HT4-like receptors", pages 619-622, see the whole article (cited in the application)
- Br. J. Pharmol., vol. 100, no. 4, August 1990, Macmillan Press Ltd, A.J. Kaumann et al.: "A 5-hydroxytryptamine receptor in human atrium", pages 879-885, see the whole article (cited in the application)
- J. Mol. Cell. Cardiol, vol. 21, suppl.2, 1989, Congress Ann Arbor, Michigan, 14-18 May 1989, H. Kameda et al.: "The effects of ICS 205-930, A new 5-HT3 receptor antagonist, on responses of guinea pig atria", see abstract P8, page S188
- Naunyn-Schmiedeberg's Arch Pharmacol, vol. 339, no. 3, March 1989, Springer-Verlag, H.Berthold et al.: "Inhibition of the 5-HT-induced cardiogenic hypertensive chemoreflex by the selective 5-HT3 receper antagonist ICS205-930" pages 259-262, see summary; page 261, right-hand column, last paragraph
- J. Mol. Cell, Cardiol, suppl. 2, vol. 21, 1989, M. Kameda et al.: "Cardiac effect of novel serotonin receptor (5-HT3) antagonists in guinea pigs", see abstract 344, page S115
- Naunyn-Schmiedeberg's Arch Pharmocol, vol. 335, no. 6, June 1987, Springer-Verlag, G. Scholtysik et al.: "Evidence for inhibition by ICS 205-930 and stimulation by BRL 34915 of K+ conductance in cardiac muscle", pages 692-696, see the whole article
- BR. J. PHARMACOLOGY, vol. 100, 1990, GREAT BRITAIN pages 879-885, KAUMAN et al
- DORLAND'S MEDICAL DICTIONARY 28TH EDITION, P. 121, 604, 1591, W.B. SAUNDERS, USA
- BR. J. PHARMACOLOGY, vol.100, 1993, GREAT BRITAIN pages 108, 245 - , KAUMAN ET AL
- BR. J. PHARMACOLOGY, vol. 100, 1990, GREAT BRITAIN pages 879-885, KAUMAN et al

## Description

The present invention relates to medicaments for use in the treatment of atrial fibrillation and the prevention of stroke.

Compounds which antagonise the effects of 5-HT₄ receptors in the central nervous system, and gastrointestinal system are known in the art (see for example European Journal of Pharmacology, 146 (1988), 187-88, Naunyn-Schmiedeberg's Arch. Pharmacol. (1989) 340:403-410) and Br. Journal of Pharmacology 96:247p. 1989).

In addition, 5-HT receptors which may resemble those known to exist in the CNS, have been identified in the human atrium [Br. Journal of Pharmacology, 98, 664p, (1989) and Br. Journal of Pharmacology, 100, 879-885 (1990)]. These "5-HT₄-like" receptors located in the atrium are hereinafter referred to as cardiac 5-HT₄ receptors.

However, in spite of these disclosures there is no indication as to the potential clinical usefulness of compounds which block cardiac 5-HT₄ receptors.

It has now been found that the compound (3-α-tropanyl)-1H-indazole-3-carboxylic acid ester antagonises cardiac 5-HT₄ receptors and is expected, as a consequence, to be of use in the treatment of atrial fibrillation.

The present invention therefore provides the use of a cardiac 5-HT₄ receptor antagonist in the manufacture of a medicament for use in the treatment of atrial fibrillation.

Cardiac arrhythmias have been associated with symptomatic cerebral embolism. Cerebral embolism is the most common cause of ischaemic stroke and the heart, the most common source of embolic material. Of particular concern is the frequency of embolism associated with atrial fibrillation (Harrison's Principles of Internal Medicine, 11th Edition). Up to 9% of individuals over the age of 60 have atrial fibrillation (Sirna et al. 1990, Stroke 21; 14-22). Atrial fibrillation in combination with rheumatic valvular heart disease is associated with a 17-fold increased risk of stroke, whereas chronic atrial fibrillation without rheumatic valvular heart disease is associated with a 5-fold increased risk of stroke. Only arrhythmias which encourage atrial stasis, such as atrial fibrillation and atrial disorders cause cerebral and systemic embolism. Thus, specific cardiac 5-HT₄ receptor antagonists which prevent atrial fibrillation and other atrial arrhythmias associated with 5-HT, would also be expected to reduce occurrence of stroke.

In a further aspect of the invention therefore, said treatment may be for the prevention of stroke.

As indicated above, the compound (3-α-tropanyl)-1H-indazole-3-carboxylic acid ester has been found to be a cardiac 5-HT₄ receptor antagonist and is expected to be of use in the treatment of the conditions hereinbefore defined.

The cardiac 5-HT₄ receptor antagonists will usually be formulated in a standard pharmaceutical composition. Said pharmaceutical composition will comprise a cardiac 5-HT₄ receptor antagonist in association with a pharmaceutically acceptable carrier. The composition can be prepared by methods well known in the art of pharmacy, for example, compounds which are active when given orally can be formulated as liquids, for example, syrups, suspensions or emulsions, tablets, capsules and lozenges.

Liquid formulations will generally consist of a suspension or solution of the compound or pharmaceutically acceptable salt in a suitable liquid carrier(s), for example, ethanol, glycerine, non-aqueous solvent, for example, polyethylene glycols, oils, or water with a suspending agent, preservative, flavouring or colouring agent.

Tablet compositions can be prepared using any suitable pharmaceutical carrier(s) routinely used for preparing solid formulations. Examples of such carriers include magnesium stearate, starch, lactose, sucrose and cellulose.

Compositions in the form of a capsule can be prepared using routine encapsulation procedures. For example, pellets containing tee active ingredient can be prepared using standard carriers and then filled into a hard gelatin capsule; alternatively, a dispersion or suspension can be prepared using any suitable pharmaceutical carrier(s), for example, agueous gums, celluloses, silicates or oils and the dispersion or suspension then filled into a soft gelatin capsule.

Typical parenteral compositions consist of a solution or suspension of the compound or pharmaceutically acceptable salt in a sterile agueous carrier or parenterally acceptable oil, for example, polyethylene glycol, polyvinyl pyrrolidone, lecithin, arachis oil or sesame oil. Alternatively, the solution can be lyophilised and then reconstituted with a suitable solvent just prior to administration.

Preferably, the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base. The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

### DATA

(3-α-Tropanyl)-1H-indazole-3-carboxylic acid ester can be prepared by the procedures described in EP-200444-A.

### PIGLET SPONTANEOUS BEATING ATRIA SCREEN

**Method** (See A. J. Kaumann, 1990, Naunyn-Schmiedeberg's Arch. Pharmacol 342, 619-622).

Piglets of either sex, 2 to 5 days old, were obtained from local farms. Usually 2 piglets were from the same litter. The piglets were anaesthetised with halothene; their hearts rapidly removed and washed free of blood with warm solution containing (mM):120 Na⁺, 5 K⁺, 2.25 Ca²⁺, 0.5 Mg²⁺, 98.5 Cl⁻, 0.5 SO₄²⁻, 34 HCO₃⁻, 1 HPO₄²⁻, 0.04 EDTA, deionised and double distilled water in glass, equilibrated with 95% O₂ and 5% CO₂.

After dissection of the right atrium in warm solution, the tissue was set up in an apparatus with a 50 ml. bath (Blinks, 1965 J. Appl. Physiol. 20, 755-757), containing the solution described above supplemented with (mM):15 Na⁺, 5 fumarate, 5 pyruvate, 5 L-glutamate, 10 glucose. Experiments were carried out at 37°C. For the study of positive chronotropic effects, the spontaneously beating right atrium was suspended at a resting tension just sufficient for measurable development of tension (to avoid tachycardia induced by stretching the sinoatrial node - Blinks, 1956 Amer. J. Physiol. 186, 299-303). The tissue was attached to a Swema SG4-45 strain gauge transducer by means of a stainless steel wire and force recorded on a Watanabe polygraph.

To avoid possible indirect β-adrenoceptor-mediated effects due to release of noradrenaline all experiments were carried out in the presence of 400 nM (±)-propranolol (about 100 x K_{B} for (±)-propranolol). To reduce tissue capture of 5-HT, all experiments were carried out in the presence of 6 µM cocaine; cocaine potentiates the effects of 5-HT on human atrium [Kaumann et al., Br. J. Pharmacol. 100, 879-885, (1990)]. To decrease oxidation of 5-HT the physiological solution and all dilutions of 5-HT contained 0.2 mM ascorbate.

A single cumulative concentration-effect curve was determined by the sequential addition of 5-HT or other agonists to the bath in amounts that increased the total concentration in steps of ½ log unit. Enough time was allowed for each effect to reach equilibrium. The experiments were terminated by the administration of a saturating concentration of (-)-isoprenaline (0.2 mM). The time of incubation with an antagonist was at least 1 hr before a concentration-effect curve for an agonist was determined.

### Results

In the above screen, the potency of a number of known agonists was found to be similar to that published for human atrium [Kaumann et al., Br. J. Pharmacol. 100, 879-885, (1990)]. These results are consistent with the hypothesis that the 5-HT receptors of porcine sinoatrial node and of human right atrial appendage are the same.

The preliminary screening results shown in Table 1 indicate that (3-α-tropanyl)-1H-indazole-3-carboxylic acid ester is a competitive antagonist of cardiac 5-HT₄ receptors.

**TABLE 1**

| **Comparison of blocking potencies of antagonists on right atrial preparations** | | | | |
|---|---|---|---|---|
| | Porcine (rate) | | Human (force) | |
| | pK | n | pK | n |
| (3-α-tropanyl)-1H-indazole-3-carboxylic acid ester | 7.1 | 6 | ND | - |
| Granisetron (EP-200444-A) | ineffective (1 µM) | 3 | ineffective (20 µM) | 2 |

| | | | | |
|---|---|---|---|---|
| pK = -log M equilibrium dissociation constant | | | | |
| ND = not determined | | | | |
| n = number of individuals | | | | |

## Claims

1. Use of a cardiac 5-HT₄ receptor antagonist in the manufacture of a medicament for use in the treatment of atrial fibrillation.

2. A use according to claim 1 wherein the treatment is for the prevention of stroke.

3. A use according to claim 1 or 2 in which the 5-HT₄ receptor antagonist is (3-α-tropanyl)-1H-indazole-3-carboxylic acid ester.

## Patentansprüche

1. Verwendung eines cardialen 5-HT₄ Rezeptor-Antagonisten zur Herstellung eines Arzneimittels zur Verwendung bei der Behandlung von Vorhofflimmern.

2. Verwendung nach Anspruch 1, wobei die Behandlung die Vorbeugung von Schlaganfällen umfaßt.

3. Verwendung nach Anspruch 1 oder 2, wobei der 5-HT₄ Rezeptor-Antagonist der (3-α-tropanyl)-1H-indazol-3-carbonsäureester ist.

## Revendications

1. Utilisation d'un antagoniste du récepteur 5-HT₄ cardiaque dans la production d'un médicament destiné à être utilisé dans le traitement de la fibrillation auriculaire.

2. Utilisation suivant la revendication 1, dans laquelle le traitement est destiné à la prévention de l'ictus.

3. Utilisation suivant la revendication 1 ou 2, dans laquelle l'antagoniste du récepteur 5-HT₄ consiste en ester d'acide (3-α-tropanyl)-1H-indazole-3-carboxylique.
